# EUROPEAN PATENT APPLICATION

(11) **EP 4 147 741 A1**
(43) Date of publication of application: **15.03.2023**
(21) Application number: 21939749.4
(22) Date of filing: 20.08.2021
(51) Int. Cl.: A61M 25/01, A61F 2/24, A61F 2/95

(54) **CONTROL HANDLE, BENDING CONTROL LINE AND IMPLANT DELIVERY DEVICE**

(30) Priority: 15.07.2021 CN 202110801252
(71) Applicant: Shanghai Trulive Medtech Co., Ltd, Shanghai 201206 (CN); Jiangsu Trulive Medtech Co., Ltd, Rudong County Nantong Jiangsu 226400 (CN)
(72) Inventor: SUN, Yunyan, Shanghai 201206 (CN); ZHANG, Wei, Shanghai 201206 (CN); ZHAO, Jing, Shanghai 201206 (CN); XU, Hongwei, Shanghai 201206 (CN); XU, Haoran, Shanghai 201206 (CN)
(74) Representative: Nordmeyer, Philipp Werner
(86) International application number: PCT/CN2021/113829
(87) International publication number: WO 2022/233104

(57) **Abstract**

A control handle (1), a control wire (21) and an implant (3) delivery device are disclosed. The control handle (1) includes a basic part (11) and a rotary part (12). The basic part (11) is configured to connect a flexible catheter (22) in a catheter assembly (2) and has an axial direction that is as same as an axial direction of the flexible catheter (22). The rotary part (12) is rotatably coupled to the basic part (11) along a radial direction of the basic part (11), and an rotational axis of the rotary part (12) is not coplanar with an axis of the basic part (11). The rotary part (12) is fixedly coupled to a proximal end of the control wire (21) and is configured to rotate to cause the control wire (21) to be wound thereon. The rotational axis of the rotary part (12) is not coplanar with the axis of the basic part (11), as a result of rotation of the rotary part (12), the control wire (21) is wound thereon, enabling bending control. In this way, the control handle (1) is allowed to have a reduced length, leading to increased structural compactness. Further, the rotary part (12) on which the control wire (21) is wound can be resized to enable adjustment of transmission ratio and hence bending speed.

## Description

### TECHNICAL FIELD

The present invention relates to the field of medical devices and, in particular, to a control handle, a control wire and an implant delivery device

### BACKGROUND

The heart has four chambers: the left atrium and left ventricle on the left side of the heart; and the right atrium and right ventricle on the right side of the heart. In addition, there are ventricular inflow tracts between the atria and ventricles, the left ventricular outflow tract consisting of the left ventricle and aorta and the right ventricular outflow tract consisting of the right ventricle and pulmonary artery. There are also "one-way valves" in the ventricular inflow and outflow tracts. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent the backward flow of blood. Defects in any one of these valves can induce hemodynamic changes and functional abnormalities in the heart and are called valvular heart disease.

With the development of social economy and the aging of population, the incidence of valvular heart disease has increased significantly. Studies have shown that this figure has reached up to 13.3% among those 75 years or older. Surgical treatment remains first choice for patients with severe valvular disease. However, for those with advanced ages, complications in multiple organs, a history of thoracotomy or poor heart functions, the surgical approach is associated with high risk and high mortality or even precludes some patients. Transcatheter valve replacement/repair has attracted a lot of attention amongst academics and researchers thanks to its advantages such as no need for thoracotomy, minor trauma and rapid recovery.

Due to the anatomical complexity of the human body, placement accuracy is particularly important for interventional surgery. In order to deliver implants along various vascular paths accurately to target sites, delivery devices with controllable flexibility are usually used.

Typically, controllable flexibility for a catheter relies on a control wire embedded alongside the catheter. The control wire is welded to a pull ring arranged at a distal end of the catheter, and proximally coupled to a handle, as well, a slider is set in handle. In this way, through sliding the slider to move, the control wire can be pulled to bend the flexible catheter in different shape. However, this conventional technique cannot control bending speed, and slider for driving the control wire makes the handle have to be in large size, which leads to unsatisfactory overall structural compactness of the handle.

### SUMMARY OF THE INVENTION

It is an object of the present invention to overcome the above-described problems with the conventional delivery devices, i.e., a lack of bending speed control and structural incompactness, by presenting a control handle, a control wire and an implant delivery device.

The above object is attained by a control handle for an implant delivery device according to the present invention, which comprises basic part and rotary part.

The basic part is connected to a flexible catheter in a catheter assembly and has an axial direction that is as same as an axial direction of the flexible catheter.

The rotary part is rotatably coupled to the basic part, and a rotational axis of the rotary part is not coplanar with an axis of the basic part. The rotary part is fixedly coupled to a proximal end of the control wire and is configured to rotate to cause the control wire to be wound thereon.

Optionally, in the control handle, the rotary part may comprise a manipulation portion and a winding portion that are sequentially connected along an axial direction of the rotary part, wherein the manipulation portion has a radial outer size greater than a radial outer size of the winding portion, and wherein the winding portion is configured for winding of the control wire thereon.

Optionally, in the control handle, the winding portion may comprise at least two axial shaft sections with different outer diameters.

Optionally, in the control handle, the rotary part may comprise a wire securing member, wherein the rotary part is fixedly coupled to a proximal end of the control wire through the wire securing member.

Optionally, in the control handle, the wire securing member may comprise a wire anchor having a first external thread, wherein the rotary part may comprise an axial blind bore and a radial wire aperture, wherein the blind bore is in communication with the wire aperture, wherein the blind bore has a first internal thread engageable with the first external thread, wherein the blind bore is configured for insertion of the proximal end of the control wire therein through the wire aperture, and wherein the wire anchor is configured to be screwed into the blind bore, and to press and secure the control wire.

Optionally, in the control handle, the rotary part may comprise a self-locking portion, furtherly, the control handle configured with a locking member to apply a resistive torque to the self-locking portion to limit a rotation of the rotary part when a rotating torque is smaller than the resistive torque.

Optionally, in the control handle, the self-locking portion may comprise a plurality of grooves arranged circumferentially about the rotational axis of the rotary part, wherein the locking member comprises a spring plunger comprising a fixation member, an elastic element and a stop ball, wherein the stop ball is coupled to the fixation member by the elastic element, wherein the fixation member is fixedly coupled to the basic part, wherein the stop ball is brought into abutment against a corresponding groove by an elastic force provided by the elastic element, thereby applying the resistive torque to the self-locking portion.

Optionally, in the control handle, the rotary part may comprise a threaded portion having a second external thread, wherein the basic part has a second internal thread engageable with the second external thread, wherein the rotary part is threadedly connected to the basic part by the threaded portion, and wherein a rotation of the rotary part with respect to the basic part is converted into an axial translation of the rotary part with respect to the basic part through the second external thread and the second internal thread.

Optionally, in the control handle, the basic part may comprise a hollow catheter fixture oriented in the axial direction of the basic part, wherein the catheter fixture is configured for insertion of a proximal end of the flexible catheter therein, and wherein the catheter fixture comprises a radial wire slot configured for a passage of the control wire therethrough.

Optionally, the control handle may further comprise a limiting shell , wherein the wire slot extends in an axial direction of the catheter fixture to a distal end of the catheter fixture, wherein the catheter fixture is provided at the distal end thereof with a third external thread, and the limiting shell has a third internal thread engageable with the third external thread, and wherein the limiting shell is threadedly connected to the catheter fixture.

The above object is also attained by a control wire for the control handle as defined above according to the present invention. The control wire comprises a first traction wire segment and a second traction wire segment, wherein the first traction wire segment is fixedly coupled at a distal end thereof to a proximal end of the second traction wire segment, wherein the first traction wire segment is more flexible than the second traction wire segment, wherein the first traction wire segment is fixedly coupled at a proximal end thereof to the rotary part, wherein the first traction wire segment is configured to be wound on the rotary part.

Optionally, in the control wire, the first traction wire segment may be implemented as a polymeric wire segment and the second traction wire segment is implemented as a metallic wire segment.

The above object is also attained by an implant delivery device according to the present invention, which comprises the control handle as defined above and the catheter assembly. The catheter assembly comprises the control wire as defined above and the flexible catheter. The distal end of the control wire is inserted within the catheter and is fixedly coupled to a distal end of the flexible catheter.

In summary, the present invention provides a control handle, a control wire and an implant delivery device. The control handle includes a basic part and a rotary part. The basic part is configured to connect a flexible catheter in a catheter assembly and has an axial direction that is as same as an axial direction of the flexible catheter. The rotary part is rotatably coupled to the basic part along a radial direction of the basic part, and an axis of rotation of the rotary part is not coplanar with an axis of the basic part. The rotary part is fixedly coupled to a proximal end of the control wire and is configured to rotate to cause the control wire to be wound thereon.

In this arrangement, since the axis of rotation of the rotary part is not coplanar with the axis of the basic part, as a result of rotation of the rotary part, the control wire is wound on the rotary part, enabling bending control. In this way, the control handle is allowed to have a shorter length, leading to increased structural compactness. Further, the rotary part on which the control wire is wound can be resized to enable adjustment of transmission ratio and hence bending speed, which can better address the needs of different applications.

### BRIEF DESCRIPTION OF THE DRAWINGS

Those of ordinary skill in the art would appreciate that the following drawings are presented merely to enable a better understanding of the present invention rather than to limit the scope thereof in any sense. In the drawings:
Fig. 1 is a schematic overview of a control handle according to an embodiment of the present invention;
Fig. 2 is a partially exploded view of the control handle of Fig. 1;
Fig. 3 is a schematic diagram illustrating the control handle according to an embodiment of the present invention;
Fig. 4 is a schematic illustration of a catheter fixture according to an embodiment of the present invention;
Fig. 5 schematically illustrates a preferred example of a rotary part according to an embodiment of the present invention;
Fig. 6 schematically illustrates another preferred example of the rotary part according to an embodiment of the present invention;
Fig. 7 schematically illustrates a self-locking portion and a locking member according to an embodiment of the present invention;
Fig. 8 is a schematic illustration of a control wire according to an embodiment of the present invention;
Fig. 9 is a schematic illustration of a delivery device according to an embodiment of the present invention; and
Fig. 10 is a schematic illustration of a distal end portion of a catheter assembly according to an embodiment of the present invention.

In these figures,
1, a control handle; 11, a basic part; 110, a base body; 111, a catheter fixture; 112, a wire slot; 113, an adhesive filling aperture; 114, a third external thread; 115, a protrusion; 116, a stop groove; 117, a circlip; 118, an air evacuation aperture; 12, a rotary part; 121, a manipulation portion; 122, a winding portion; 123, a threaded portion; 124, a wire anchor; 125, a wire aperture; 126, a transition section; 127, a self-locking portion; 128, a groove; 129, a limiting member; 13, a shell; 131, a limiting shell; 14, a spring plunger; 141, a fixation member; 142, a stop ball;
2, a catheter assembly; 21, a control wire; 211, a first traction wire segment; 212, a second traction wire segment; 22, a flexible catheter; 221, a balloon; 222, an outer catheter; 223, a catheter holder; 2231, a filling port; 2232, a guidewire exit; 23, a balloon catheter; 231, a tip; 232, a balloon supporter ; 233, an inner catheter; 24, a control wire exit;
3, an implant.

### DETAILED DESCRIPTION

Objects, advantages and features of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear description of the embodiments. In addition, the structures shown in the figures are usually partial representations of their actual counterparts. In particular, as the figures would have different emphases, they are sometimes drawn to different scales.

As used herein, the singular forms "a", "an" and "the" include plural referents, and the term "or" is generally employed in the sense of "and/or", "several" of "at least one", and "at least two" of "two or more than two". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. The term "proximal end" usually refers to an end closer to an operator, and the term "distal end" usually refers to an end closer to a patient or lesion. The terms "one end" and "the other end", or "proximal end" and "distal end", are generally used to refer to opposite end portions including the opposite endpoints, rather than only to the endpoints. As used herein, the terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two components. Furthermore, when an element is referred herein to as being disposed on another element, it is typically only meant that the two elements are connected, coupled, mated or geared to each other either directly or indirectly with the presence of intervening element(s). Such reference should not be interpreted as indicating or implying any relative spatial relationship between the two elements. That is, the referenced element may be positioned inside, outside, above, beneath, beside or in any other spatial relationship to the other element, unless the context clearly specifies. Those of ordinary skill in the art can understand the specific meanings of the above-mentioned terms herein, depending on their context.

It is an object of the present invention to overcome the above-described problems with the conventional delivery devices, i.e., a lack of bending speed control and structural incompactness, by presenting a control handle, a control wire and an implant delivery device. The invention will be described below with reference to the accompanying drawings.

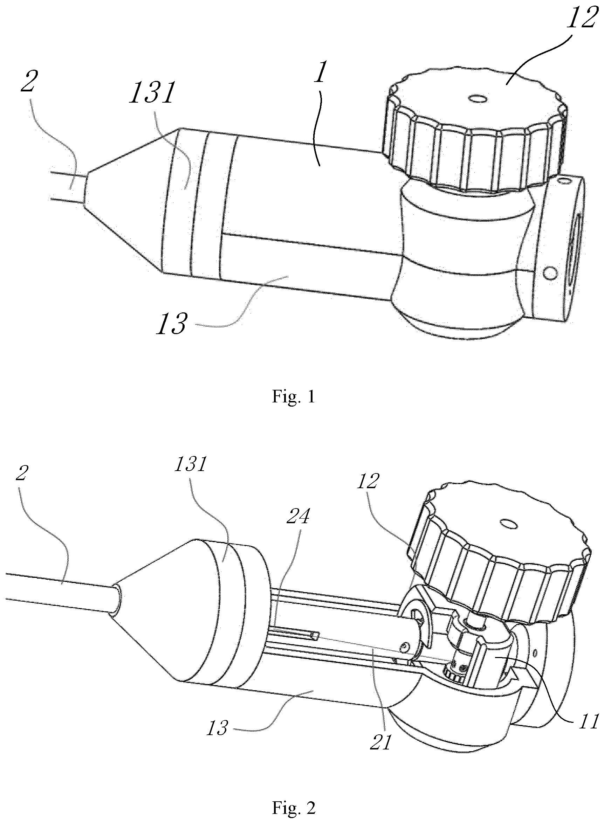

Referring to Figs. 1, 2, 9 and 10, in embodiments of the present invention, there is provided a delivery device for delivering an implant 3, which may be, for example, a prosthetic heart valve. The delivery device includes a control handle 1 and a catheter assembly 2. The catheter assembly 2 includes a control wire 21 and a flexible catheter 22. A distal end of the control wire 21 is inserted in the flexible catheter 22 and secured to a distal end of the flexible catheter 22, for example, by welding. A middle section of the control wire 21 may be disposed within the flexible catheter 22, e.g., embedded in a wall of the flexible catheter 22, and the control wire 21 may lead out of the wall of the flexible catheter 22 through a control wire exit 24 around a proximal end of the flexible catheter 22. After leading out of the flexible catheter 22 through the control wire exit 24, a proximal end of the control wire 21 is coupled to the control handle 1. With this arrangement, the control handle 1 cause the flexible catheter 22 to bend through controlling axial movement of the control wire 21 so as to achieve the bending control. The structure and function of the flexible catheter 22 are similar to those of conventional catheters known to those skilled in the art and, therefore, need not be described in further detail herein.

Optionally, the catheter assembly 2 may further include a balloon catheter 23 disposed within the flexible catheter 22. In an alternative exemplary embodiment, the balloon catheter 23 includes a tip 231, balloon supporters 232 and an inner catheter 233 that are sequentially connected in the direction from a distal end to a proximal end thereof. The flexible catheter 22 includes a balloon 221, an outer catheter 222 and a catheter holder 223 that are sequentially connected in the direction from the distal end to the proximal end thereof. A distal end of the balloon 221 is sealingly connected to a distal end of the balloon supporters 232. The balloon 221 is collapsible or foldable. Moreover, it is dilatable when a fluid (e.g., a contrast fluid) is filled therein. A proximal end of the inner catheter 233 is sealingly connected to the catheter holder 223. The inner catheter 233 comprises a lumen for passage of a guidewire therethrough. At a proximal end of the catheter holder 223, there are provided a filling port 2231 and a guidewire exit 2232. The guidewire exit 2232 is preferably arranged in an axial direction of the inner catheter 233, while the filling port 2231 may be arranged at an angle with respect to the axial direction of the inner catheter 233, thus forming a branch. A clearance formed between the inner catheter 233 and the outer catheter 222 is brought into communication with the filling port 2231 to allow the fluid to be filled into or discharged from the balloon 221 through the clearance between the inner catheter 233 and the outer catheter 222. Before the fluid is filled into the balloon 221, the balloon 221 is in a collapsed or folded configuration, the implant 3 can be crimped over, and move with, the balloon 221. Upon the implant 3 being advanced to a target site, the fluid may be filled into the balloon 221 through the filling port 2231 to expand the balloon 221 and hence the implant 3 to a predetermined size.

In order to address the problem sought to be solved by the present invention, referring to Figs. 1 to 3, according to embodiments of the present invention, the control handle 1 includes a basic part 11 and a rotary part 12. The basic part 11 is configured to connect the flexible catheter 22 in the catheter assembly 2 and has an axial direction that is the same as an axial direction of the flexible catheter 22. It is to be understood that, since the flexible catheter 22 is flexible and bendable, its axial direction would vary when it bends. However, here, the axial direction of the flexible catheter 22 shall be interpreted as an axial direction of a section of the flexible catheter 22 in direct connection with the basic part 11.

The rotary part 12 is rotatably coupled to the basic part 11 about a rotational axis which is not coplanar with an axis of the basic part 11. Preferably, the rotational axis of the rotary part 12 is perpendicular to the axis of the basic part 11. The rotary part 12 is fixedly connected to the proximal end of the control wire 21 and configured to rotate to cause the control wire 21 to be wound thereon. With this arrangement, the rotary part 12 is able to convert movement of the control wire 21 in the axial direction thereof to a circumferential movement around the rotary part 12. Accordingly, as a result of rotation of the rotary part 12, the control wire 21 will be wound on the rotary part 12, enabling bending control of the flexible catheter. In this way, the control handle 1 is allowed to have a shorter length, leading to increased structural compactness. Further, the rotary part 12 on which the control wire 21 is wound may be resized to enable adjustment of transmission ratio and hence bending speed, which can better address the needs of different applications.

Preferably, the rotary part 12 includes a manipulation portion 121 and a winding portion 122 that are connected together along the axial direction thereof,. A radial outer size of the manipulation portion 121 is greater than a radial outer size of the winding portion 122. The winding portion 122 is configured for winding of the control wire 21 thereon. It is to be noted that, here, the "radial outer size" of the manipulation portion 121 refers to maximum radial size in a radial direction of the rotary part 12. In an alternative embodiment, the manipulation portion 121 has a circular peripheral circumference. In this case, the radial outer size of the manipulation portion 121 refers to its outer diameter. If the peripheral circumference of the manipulation portion 121 is polygonal, then the radial outer size of the manipulation portion 121 will refer to a diameter of a circle circumscribed around the polygon. The same applies to the radial outer sizes of the components described below. The manipulation portion 121 is configured to be gripped and rotated by an operator. When the operator applies an external force on the manipulation portion 121, a rotating torque will be created to drive the entire rotary part 12 to rotate about its own rotational axis. Therefore, in order to provide a sufficient rotating torque, the manipulation portion 121 is desired to have a large enough radial outer size. In the exemplary embodiment shown in Figs. 1 and 2, the manipulation portion 121 is a toothed wheel with multiple grooves, which can improve friction and facilitate the operator's manipulation. The winding portion 122 is preferred to have a circular peripheral circumference, which can facilitate winding of the control wire 21 thereon.

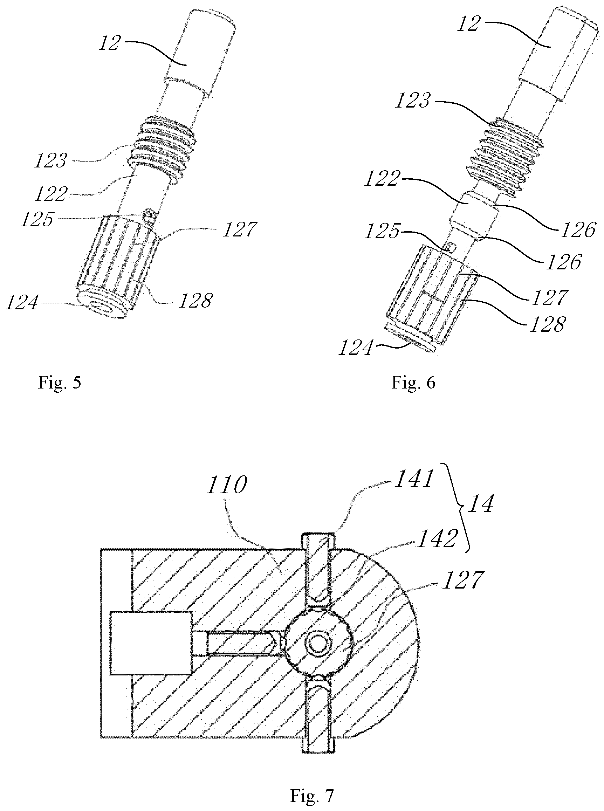

Referring to Figs. 5 and 6, the rotary part 12 may optionally include a wire securing member. In this case, the proximal end of the control wire 21 may be secured to the rotary part 12 by the wire securing member. In an alternative embodiment, the wire securing member includes a wire anchor 124 having a first external thread. The rotary part 12 comprises an axial blind bore and a radial wire aperture 125 in communication with the blind bore. The blind bore has a first internal thread engageable with the first external thread and is configured to allow the proximal end of the control wire 21 to be inserted therein through the wire aperture 125. The wire anchor 124 is configured to be screwed into the blind bore, and to press and secure the control wire 21. It would be appreciated that the wire anchor 124 is merely one example of the wire securing member and does not imply any limitation to the type of wire securing member. In other non-limiting embodiments, those skilled in the art may choose another type of wire securing member, such as a securing hook, a securing ring or the like.

As shown in Figs. 1 and 2, the control handle 1 may further include a shell 13 configured to accommodate and protect the basic part 11 and winding portion 122, with the manipulation portion 121 being arranged out of the shell 13. The shell 13 is shaped to allow easy gripping.

Referring to Figs. 2 to 4, the basic part 11 may optionally include a hollow catheter fixture 111, which extends in the axial direction of the basic part 11 and configured to connect the flexible catheter 22. The catheter fixture 111 comprises in its wall a wire slot 112 configured for passage of the control wire 21 therethrough. In an alternative exemplary embodiment, both the catheter fixture 111 and the flexible catheter 22 are round tubes, and an inner diameter of the catheter fixture 111 matches an outer diameter of the flexible catheter 22. A proximal end portion of the flexible catheter 22 may be inserted into the catheter fixture 111 and attached to the wall thereof by an adhesive. Preferably, the catheter fixture 111 may further comprise in its wall an adhesive filling aperture 113, through which the adhesive can be filled into a gap between the flexible catheter 22 and the catheter fixture 111 to enable the attachment. As shown in Fig. 2, the proximal end of the control wire 21 is inserted together with the flexible catheter 22 into the catheter fixture 111, pulled out of the catheter fixture 111 through the wire slot 112 and secured to the winding portion 122. It would be appreciated that, since the rotational axis of the rotary part 12 is not coplanar with an axis of the catheter fixture 111, a section of the control wire 21 pulled out of the wire slot 112 is generally oriented at an angle with respect to the axis of the catheter fixture 111. In order to facilitate assembly, the wire slot 112 may have a length along an axial direction of the catheter fixture 111, which is sufficient to allow desirable placement of the control wire 21.

Preferably, wire slot 112 extends in the axial direction of the catheter fixture 111 to a distal end thereof. Accordingly, a distal end portion of the catheter fixture 111 has an open C-shaped cross-section. Additionally, a third external thread 114 may be provided around the distal end of the catheter fixture 111, and the shell 13 may have a limiting shell 131 having a third internal thread engageable with the third external thread 114. In this way, the limiting shell 131 can be threadedly connected to the catheter fixture 111. When this threaded connection of the limiting shell 131 with the catheter fixture 111 is being established, due to the open C-shaped cross-section, the distal end portion of the catheter fixture 111 will deform inwardly as it is being compressed by the limiting shell 131. As a result, an interior surface of the catheter fixture 111 will tightly abut against an exterior surface of the flexible catheter 22, resulting in more reliable connection between the flexible catheter 22 and the catheter fixture 111.

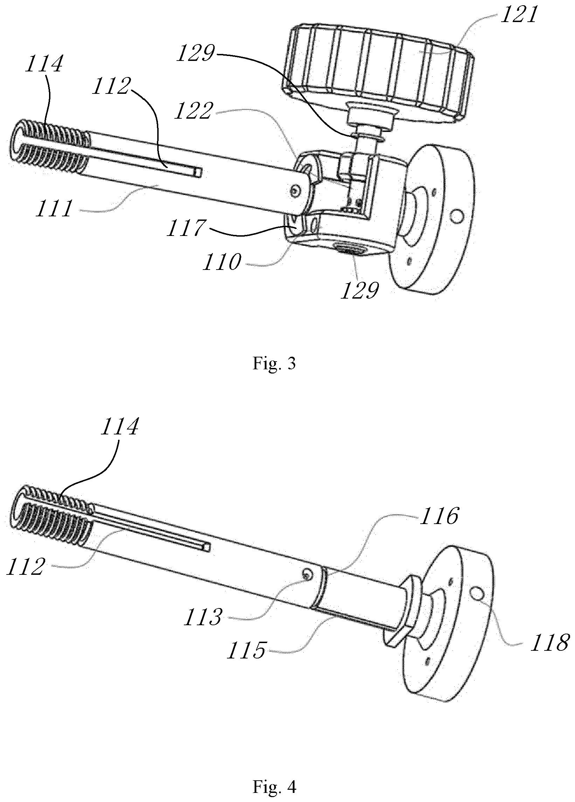

With continued reference to Fig. 3, in an alternative embodiment, the basic part 11 includes a base body 110 connected with the catheter fixture 111 in a matched manner. Optionally, the base body 110 may comprise a through bore axially extending therethrough. The through bore may have an inner diameter that matches an outer diameter of the catheter fixture 111, and the catheter fixture 111 may be configured to be inserted within the through bore. Preferably, the catheter fixture 111 has a radial protrusion 115, and the through bore of the base body 110 has a recess matching the protrusion 115. More preferably, both the protrusion 115 and the recess extend axially. Once the catheter fixture 111 is inserted into the through bore of the base body 110, the protrusion 115 will be received in the recess, thereby limiting circumferential movement of the catheter fixture 111 with respect to the base body 110. The catheter fixture 111 may further have a circumferentially arranged stop groove 116, and after the catheter fixture 111 is inserted into the through bore of the base body 110, a circlip 117 may be fitted in the stop groove 116 to limit axial movement of the catheter fixture 111 with respect to the base body 110. This enables the catheter fixture 111 to be assembled with the base body 110 in such a manner that they stay stationary with respect to each other. The catheter fixture 111 may further have an air evacuation aperture 118, which is brought into communication with the clearance between the inner catheter 233 and the outer catheter 222 to enable evacuation of air from between the inner catheter 233 and the outer catheter 222.

Referring to Figs. 5 and 6, in conjunction with Fig. 2, optionally, the rotary part 12 may include a threaded portion 123 having a second external thread, and the basic part 11 may have a second internal thread engageable with the second external thread. In this way, the rotary part 12 can be threadedly connected to the basic part 11 through the threaded portion 123, and rotation of the rotary part 12 with respect to the basic part 11 can be converted into axial translation of the rotary part 12 with respect to the basic part 11 by virtue of the second external and internal threads. In an exemplary embodiment, in addition to engaging the catheter fixture 111, the base body 110 also serves to hold a rotary shaft of the rotary part 12. It would be appreciated that, once the rotary part 12 is threadedly connected to the base body 110, the rotary part 12 will translate with respect to the base body 110 when it is rotating with respect thereto. Since rotation of the rotary part 12 will cause the control wire 21 to be wound on the winding portion 122, thread pitches of the second external and internal threads may be designed to match an outer diameter of the control wire 21 (i.e., the first traction wire segment, as detailed below) in order to allow the control wire 21 to be wound on the winding portion 122 in a desired manner without overlapping.

Preferably, the rotary part 12 further includes two axially spaced limiting members 129, such as circlips, which are raised over the main portion of the rotary part 12. When the rotary part 12 axially translates with respect to the base body 110 to predetermined positions, as a result of its own rotation, the limiting members 129 will come into abutment against the base body 110 and thus the rotary part 12 cannot axially move any more. In this way, axial movement of the rotary part 12 and hence displacement of the control wire 21 is limited within a predetermined range.

Optionally, the rotary part 12 may include a self-locking portion 127, and the control handle 1 may further include a locking member configured to apply a resistive torque to the self-locking portion 127 to prevent the rotary part 12 from rotate when the rotating torque is smaller than the resistive torque. Referring to Fig. 7, in an exemplary embodiment, the self-locking portion 127 includes multiple grooves 128 distributed circumferentially about the rotational axis of the rotary part 12, and the locking member includes a spring plunger 14 including a fixation member 141, an elastic element (not shown) and a stop ball 142. The stop ball 142 is connected to the fixation member 141 by the elastic element, and the fixation member 141 is fixed to the basic part 11 (e.g., to the base body 110). The stop ball 142 is brought into abutment against one of the grooves 128 by an elastic force provided by the elastic element, thus by applying the resistive torque to the self-locking portion 127. Optionally, in the spring plunger 14, the fixation member 141 may be substantially a cylindrical sleeve closed at one end and open at the other end, and the elastic element may be, for example, a spring disposed within the fixation member 141 and connected to the fixation member 141 at one end and to the stop ball 142 at the other end. The stop ball 142 is brought into abutment against one of the grooves 128 by an elastic force from the elastic element, thus providing the resistive torque about the rotational axis of the rotary part 12. In order to cause the rotary part 12 to rotate, the resistive torque must be overcome to push the stop ball 142 toward the spring plunger 14. Once the rotating torque exerted on the rotary part 12 is smaller than the resistive torque, rotation of the rotary part 12 will be stopped by the spring plunger 14, and the rotary part 12 will be self-locked. The self-locking portion 127 and the locking member help enhance rotational accuracy of the rotary part 12 and bending control accuracy. Preferably, the locking member includes more than two (e.g., three) spring plungers 14, which are arranged at angles with respect one another. It is to be understood that the cooperation between the grooves 128 and the spring plunger 14 is only an example of cooperation between the self-locking portion 127 and the locking member, without limiting the structures of the self-locking portion 127 and the locking member in any way. Those skilled in the art can reasonably modify and configure the self-locking portion 127 and the locking member based on the prior art (e.g., they may be alternatively implemented as magneto resistive components), without departing from the scope of the present invention.

As shown in Fig. 5, in one preferred example, the winding portion 122 includes only one cylindrical shaft section with a constant outer diameter. In this case, if the diameter of the winding portion 122 is denoted by D, then a distance L the control wire 21 travels along its own axial direction as a result of the rotary part 12 rotating one revolution will satisfy: L=π^{∗}D. Therefore, the bending speed may be adjusted by changing the diameter D of the winding portion 122. Those skilled in the art can select a suitable diameter D of the winding portion 122, as practically required.

As shown in Fig. 6, in one preferred example, the winding portion 122 includes at least two axial shaft sections with different outer diameters. In this case, depending on the outer diameter of the shaft section on which the control wire 21 is being wound, rotation of the rotary part 12 by one revolution will result in different amounts of axial displacement of the control wire 21. As a result, different moving speeds of the control wire 21 and hence different bending speeds can be provided at the same rotational speed of the rotary part 12 within the same delivery process. In this way, applications requiring different bending speeds can be addressed, and rapid bending and accurate locating can be achieved. In practical use, the rotary part 12 may be maintained at a substantially constant rotational speed, and the shaft sections with different outer diameters may be configured and arranged to achieve bending speed adjustments.

In the exemplary embodiment of Fig. 6, the winding portion 122 includes two axial shaft sections with different outer diameters, which are connected together by a transition section 126. It is to be noted that the transition section 126 is not mandatory, and the two shaft sections with different outer diameters may also be directly connected to each other.

In some implant delivery applications, a varying bending speed may be required. For example, during delivery of a heart valve prosthesis, slow bending may be desirable before the prosthesis reaches the aortic arch. However, in order to advance the prosthesis through the aortic arch, rapid bending may be more favored. After this, a slower bending speed may be again desired. Correspondingly, the winding portion 122 may include three axial shaft sections with outer diameters respectively of 2.5 mm, 4.0 mm and 2.5 mm. Adjacent shaft sections are preferably connected by transition sections 126. Axial lengths of the three shaft sections may be configured as practically required. In use, the heart valve prosthesis may be crimped over a middle section of the balloon 221 using a crimper before the procedure starts, and it is ensured that both ends of the heart valve prosthesis are situated between the two balloon supporters 232. A distal end portion of the catheter assembly 2 may be then advanced within the femoral artery, so as to deliver the heart valve prosthesis to distal end. Immediately before the heart valve prosthesis reaches the aortic arch, the manipulation portion 121 is rotated to commence bending control during advancement. The bending control undergoes the three successive bending phases: slow, rapid and slow. The prosthesis is then advanced to a target site. Then a predetermined amount of a diluted contrast fluid may be filled into the balloon via the filling port 2231 with the aid of a balloon pump to expand the heart valve prosthesis to a predetermined size.

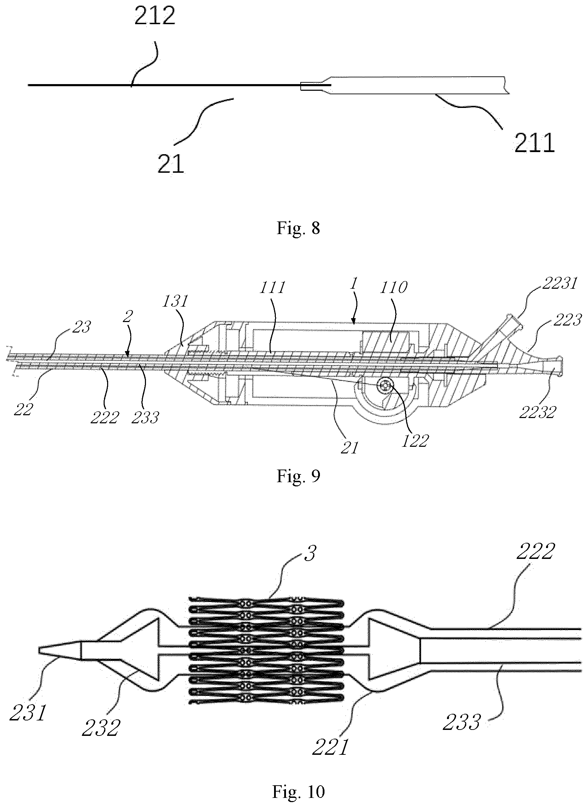

The inventors have found that, although the control handle 1 is allowed to have a reduced overall size by winding the control wire 21 on the rotary part 12, when a metal wire, such as a stainless steel wire, is chosen as the control wire, as is commonly practice in existing delivery devices, the winding may cause fatigue or even breakage of the wire. In view of this, in embodiments of the present invention, there is also provided a control wire 21 including a first traction wire segment 211 and a second traction wire segment 212. A distal end (on the left side of Fig. 8) of the first traction wire segment 211 is fixedly coupled to a proximal end (on the right side of Fig. 8) of the second traction wire segment 212. The first traction wire segment 211 is more flexible than the second traction wire segment 212 and is fixedly coupled to the rotary part 12 at its proximal end. The first traction wire segment 211 is configured to be wound on the rotary part 12. In this arrangement, the more flexible first traction wire segment 211 is adapted to be wound, while the stiffer second traction wire segment 212 is connected to the flexible catheter 22 and configured for bending control. This control wire 21 with both flexible and stiff sections can effectively circumvent the problem of fatigue or even breakage caused by winding, which may arise from the use of a stainless steel wire.

Optionally, the first traction wire segment 211 may be formed of a polymeric material, which is preferred to be polyethylene. The second traction wire segment 212 may be formed of a metallic material, which is preferred to be austenitic stainless steel, a nickel-titanium alloy or the like.

Optionally, the fixed coupling of the distal end of the first traction wire segment 211 and the proximal end of the second traction wire segment 212 may be accomplished, for example, by gluing, welding or crimping.

In summary, the present invention provides a control handle, a control wire and an implant delivery device. The control handle includes a basic part and a rotary part. The basic part is configured to connect a flexible catheter in a catheter assembly and has an axial direction that is the same as an axial direction of the flexible catheter. The rotary part is rotatably coupled to the basic part along a radial direction of the basic part, and an axis of rotation of the rotary part is not coplanar with an axis of the basic part. The rotary part is fixedly coupled to a proximal end of the control wire and is configured to rotate to cause the control wire to be wound thereon. In this arrangement, since the axis of rotation of the rotary part is not coplanar with the axis of the basic part, as a result of rotation of the rotary part, the control wire is wound on the rotary part, enabling bending control. In this way, the control handle is allowed to have a shorter length, leading to increased structural compactness. Further, the rotary part on which the control wire is wound can be resized to enable adjustment of transmission ratio and hence bending speed, which can better address the needs of different applications.

It is to be noted that the foregoing several embodiments may be combined. The description presented above is merely that of some preferred embodiments of the present invention and is not intended to limit the scope thereof in any sense. Any and all changes and modifications made by those of ordinary skill in the art based on the above teachings fall within the scope as defined in the appended claims.

## Claims

1. A control handle for an implant delivery device, wherein the control handle comprises a basic part and a rotary part,
wherein the basic part is configured to connect a flexible catheter in a catheter assembly, the basic part having an axial direction that is as same as an axial direction of the flexible catheter,
wherein the rotary part is rotatably coupled to the basic part, and a rotational axis of the rotary part is not coplanar with an axis of the basic part, and wherein the rotary part is fixedly coupled to a proximal end of a control wire and is configured to rotate to cause the control wire to be wound thereon.

2. The control handle according to claim 1, wherein the rotary part comprises a manipulation portion and a winding portion that are sequentially connected along an axial direction of the rotary part, wherein the manipulation portion has a radial outer size greater than a radial outer size of the winding portion, and wherein the winding portion is configured for winding of the control wire thereon.

3. The control handle according to claim 2, wherein the winding portion comprises at least two axial shaft sections with different outer diameters.

4. The control handle according to claim 1, wherein the rotary part comprises a wire securing member, wherein the rotary part is fixedly coupled to the proximal end of the control wire through the wire securing member.

5. The control handle according to claim 4, wherein the wire securing member comprises a wire anchor having a first external thread, wherein the rotary part comprises an axial blind bore and a radial wire aperture, wherein the blind bore is in communication with the wire aperture, wherein the blind bore has a first internal thread engageable with the first external thread, wherein the blind bore is configured for an insertion of the proximal end of the control wire therein through the wire aperture, and wherein the wire anchor is configured to be screwed into the blind bore, and to press and secure the control wire.

6. The control handle according to claim 1, wherein the rotary part comprises a self-locking portion, wherein the control handle further comprises a locking member configured to apply a resistive torque to the self-locking portion to limit a rotation of the rotary part when a rotating torque is smaller than the resistive torque.

7. The control handle according to claim 6, wherein the self-locking portion comprises a plurality of grooves arranged circumferentially about the rotational axis of the rotary part, wherein the locking member comprises a spring plunger comprising a fixation member, an elastic element and a stop ball, wherein the stop ball is coupled to the fixation member by the elastic element, wherein the fixation member is fixedly coupled to the basic part, wherein the stop ball is brought into abutment against the corresponding groove by an elastic force provided by the elastic element, thereby applying the resistive torque to the self-locking portion.

8. The control handle according to claim 1, wherein the rotary part comprises a threaded portion having a second external thread, wherein the basic part has a second internal thread engageable with the second external thread, wherein the rotary part is threadedly connected to the basic part by the threaded portion, and wherein a rotation of the rotary part with respect to the basic part is converted into an axial translation of the rotary part with respect to the basic part through the second external thread and the second internal thread.

9. The control handle according to claim 1, wherein the basic part comprises a hollow catheter fixture oriented in the axial direction of the basic part, wherein the catheter fixture is configured for an insertion of a proximal end of the flexible bending catheter therein, and wherein the catheter fixture comprises a radial wire slot configured for a passage of the control wire therethrough.

10. The control handle according to claim 9, further comprising a limiting shell, wherein the wire slot extends in an axial direction of the catheter fixture to a distal end of the catheter fixture, wherein the catheter fixture is provided at the distal end thereof with a third external thread, and the limiting shell has a third internal thread engageable with the third external thread, and wherein the limiting shell is threadedly connected to the catheter fixture.

11. A control wire for the control handle according to any one of claims 1 to 10, comprising a first traction wire segment and a second traction wire segment, wherein the first traction wire segment is fixedly coupled at a distal end thereof to a proximal end of the second traction wire segment, wherein the first traction wire segment is more flexible than the second traction wire segment, wherein the first traction wire segment is fixedly coupled at a proximal end thereof to the rotary part, wherein the first traction wire segment is configured to be wound on the rotary part.

12. The control wire according to claim 11, wherein the first traction wire segment is implemented as a polymeric wire segment and the second traction wire segment is implemented as a metallic wire segment.

13. An implant delivery device, comprising the control handle according to any one of claims 1 to 10 and a catheter assembly, wherein the catheter assembly comprises the control wire according to any one of claims 11 to 12 and a flexible catheter, wherein a distal end of the control wire is inserted within the flexible catheter and is fixedly coupled to a distal end of the flexible catheter.
